# EUROPEAN PATENT APPLICATION

(11) **EP 2 495 550 A1**
(43) Date of publication of application: **05.09.2012**
(21) Application number: 10826931.7
(22) Date of filing: 29.10.2010
(51) Int. Cl.: G01N 21/78, G01N 21/64, G01N 27/416

(54) **METHOD FOR MEASUREMENT OF FLUORESCENCE INTENSITY OF VOLTAGE-SENSITIVE FLUORESCENT DYE**

(30) Priority: 30.10.2009 JP 2009251123
(71) Applicant: Daiichi Sankyo Company, Limited, Tokyo 103-8426 (JP); Keio University, Tokyo 108-8345 (JP)
(72) Inventor: HATTORI, Fumiyuki, Kobe-shi Hyogo 650-0047 (JP); FUKUDA, Keiichi, Tokyo 160-8582 (JP); SATOH, Yu-suke, Tokyo 160-8582 (JP)
(74) Representative: Vossius & Partner
(86) International application number: PCT/JP2010/069763
(87) International publication number: WO 2011/052801

(57) **Abstract**

An object of the present invention is to provide a method for increasing the change in the fluorescent intensity as emitted from potential-sensitive fluorochromes depending on a potential or ionic strength change. Another object of the present invention is to measure the changes in the activity potentials of ES cell- or iPS cell-derived cardiomyocytes that have heretofore been impossible to measure.

The present inventors screened a variety of substances and found that vitamin E has an action for increasing the sensitivity of potential-sensitive fluorochromes whereas cholesterol has an action for enhancing the fluorescent intensity of potential-sensitive fluorochromes. In addition, it has become clear that these substances can be combined in such a way that the sensitivity of a potential-sensitive fluorochrome is increased by vitamin E while at the same time its absolute fluorescent intensity is enhanced by cholesterol.

## Description

### TECHNICAL FIELD

The present invention relates to a method that uses vitamin E and/or cholesterol so as to increase the sensitivity of potential-sensitive fluorochromes that emit fluorescence in response to a potential or ionic strength change (i.e., sensitizing the fluorochromes), or a method for enhancing the fluorescent intensity of potential-sensitive fluorochromes. The present invention also relates to a method for measuring the activity potential of cultured cardiomyocytes using measurement systems that have been sensitized or intensity-enhanced by using vitamin E and/or cholesterol. The present invention further relates to a method that uses a potential-sensitive fluorochrome forming a solid phase on surfaces of a substrate so that potential or ionic strength changes of the potential-sensitive fluorochrome can be measured irrespective of whether a membrane carrier such as cells or lipid bilayered liposomes are used or not. The present invention also relates to a method for selecting a substance that increases the percent change in the potential-dependent or ionic strength change-dependent fluorescent intensity of potential-sensitive fluorochromes or which enhances their fluorescent intensity.

### BACKGROUND ART

Arrhythmia is a disease that not only lowers the quality of life of patients considerably but which also sometimes threatens their life. In the development of drugs against various diseases, it is critical to detect and avoid any side-effects (e.g., arrhythmia) that might affect the electrogenic activities of cardiomyocytes. To date, animals and animal-derived cardiomyocytes have been used for the purposes of developing antiarrhythmic drugs and testing them for any actions that might affect cardiomuscular electrogenic activities. However, on account of the species differences involved, this has not been a practically feasible method that can be used in humans (Non-Patent Document 1: Biochem Biophys Res Commun., Vol. 385, p. 497-502, 2009).

Recent developments of human ES cells and human iPS cells have demonstrated that human cardiomyocytes can be obtained by differentiating these cells. A method capable of conveniently acquiring the activity potential of human cardiomyocytes, using those cells would play an important role in drug discovery activities. Under the current circumstances, use of potential-sensitive fluorochromes is assumed to provide a method for measuring the activity potentials of such human ES cell- or iPS cell-derived cardiomyocytes; however, the problem with the analysis using potential-sensitive fluorochromes is that whether the cardiomyocytes are derived from human ES cells or human iPS cells, it has been impossible to measure their activity potentials on account of the insufficiency in the sensitivity of the potential-sensitive fluorochromes.

A further problem with the heretofore used potential-sensitive fluorochromes is that potential measurement is possible only when they are bound to membrane carriers such as cells or lipid bilayered liposomes. To be more specific, in order to measure the sensitivity of potential-sensitive fluorochromes, it has been necessary to perform an experiment after the potential-sensitive fluorochromes in an aqueous solution are processed to form a solid phase on a membrane carrier such as cells or lipid bilayered liposomes but this has necessitated the preparation of cells or lipid bilayered liposomes as a membrane carrier. Since this approach requires that lipid bilayered liposomes be prepared or cells be used as a membrane carrier, the experimental system will not only become complicated but also be affected by various intervening artifacts.

### CITATION LIST

### NON PATIENT DOCUMENTS

Non-Patent Document 1: Biochem Biophys Res Commun., Vol.385, p. 497-502, 2009

### SUMMARY OF INVENTION

### TECHNICAL PROBLEM

An object of the present invention is to provide a method for increasing the change in the fluorescent intensity emitted from a potential-sensitive fluorochrome depending on a potential or ionic strength change and/or a method for enhancing fluorescent intensity depending on the potential or ionic strength.
Another object of the present invention is to measure the changes in the activity potentials of ES cell- or iPS cell-derived cardiomyocytes that have heretofore been impossible to measure.
A further object of the present invention is to ensure that the fluorescent intensities of potential-sensitive fluorochromes or the potential-dependent quantitative changes in their fluorescent intensity can be measured conveniently without using such substances (membrane carriers) as cells or lipid bilayered liposomes.
Yet another objet of the present invention is to develop a screening method for finding out a substance that enhances the fluorescent intensities of potential-sensitive fluorochromes.

### SOLUTION TO PROBLEM

The present inventors screened a variety of substances and found that vitamin E has an action for increasing the sensitivity of potential-sensitive fluorochromes whereas both vitamin E and cholesterol have an action for enhancing the fluorescent intensity of potential-sensitive fluorochromes. In addition, it became clear that these substances can be combined in such a way that the sensitivity of a potential-sensitive fluorochrome is increased by vitamin E while at the same time its absolute fluorescent intensity is enhanced by both cholesterol and vitamin E (or the combination of cholesterol and vitamin E). Based on these findings, the present inventors have accomplished the present invention which provides a method for measuring changes in the fluorescent intensity of a potential-sensitive fluorochrome depending on the potential or ionic strength change, wherein an ionizing compound is added to a potential-sensitive fluorochrome to confer a potential or ionic strength change and vitamin E and/or cholesterol is also added to increase the potential or ionic strength change on the potential-sensitive fluorochrome.

The present inventors also demonstrated that by employing the above-described action for sensitizing potential-sensitive fluorochromes to increase their fluorescent intensity, the heretofore impossible measurement of the activity potentials of single cells such as ES cell- or iPS cell-derived cardiomyocytes could be performed, with the additional advantage of allowing measurements of changes in the activity potential of specific areas of cells. Based on these findings, the present inventors provide a method for measuring the activity potential of cultured cardiomyocytes wherein a potential-sensitive fluorochrome is bought into contact with cardiomyocytes being cultured in a culture medium, vitamin E and/or cholesterol is added to the culture medium, and changes in the fluorescent intensity of the potential-sensitive fluorochrome depending on a potential or ionic strength change are measured.

The present inventors further made a new discovery that potential-sensitive fluorochromes can be adsorbed to form a solid phase on substrate surfaces such as plastic or glass surfaces. This is based on the discovery that potential-sensitive fluorochromes are substances that can be easily adsorbed on substrate surfaces such as plastic or glass surfaces. By using the thus formed solid phase of potential-sensitive fluorochromes, the inventors successfully developed a system by which changes in potential or ionic strength can be measured very conveniently and in a consistent and highly reproducible manner even in the absence of a membrane carrier such as cells or lipid bilayered liposomes. Based on this finding, the present inventors provide a method for measuring changes the in potential or ionic strength on a potential-sensitive fluorochrome in the absence of a membrane carrier such as cells or lipid bilayered liposomes, wherein a potential-sensitive fluorochrome is immobilized on a surface of a substrate in a solution, an ionizing compound is added to the solution to confer a potential or a change in ionic strength, and changes in the fluorescent intensity of the potential-sensitive fluorochrome depending on a potential or ionic strength change are measured.

In still another mode of the invention, the present inventors, based on the discovery that by using specific substances, the sensitivity of fluorescence from potential-sensitive fluorochromes or their absolute fluorescent intensity can be increased and that changes in the potential or ionic strength on the potential-sensitive fluorochromes can also be measured irrespective of whether a membrane carrier such as cells or lipid bilayered liposomes is used or not, demonstrated the possibility of screening for substances capable of increasing the sensitivities of potential-sensitive fluorochromes or substances capable of enhancing their absolute fluorescent intensities. Based on this observation, the present inventors provide a method for selecting a substance that enhances the percent change of fluorescent intensity of a potential-sensitive fluorochrome depending on the potential or ionic strength, comprising:
(i) immobilizing a potential-sensitive fluorochrome on a surface of a substrate in a solution, adding an ionizing compound to the solution, and measuring changes in the fluorescent intensity of the potential-sensitive fluorochrome depending on a potential or ionic strength change to thereby measure a reference value for the potential or ionic strength on the potential-sensitive fluorochrome in the absence of a membrane carrier such as cells or lipid bilayered liposomes;
(ii) immobilizing a potential-sensitive fluorochrome on a surface of a substrate in a solution, adding an ionizing compound and a test substance to the solution, and measuring changes in the fluorescent intensity of the potential-sensitive fluorochrome depending on a potential or ionic strength change to thereby measure a test value for the potential or ionic strength on the potential-sensitive fluorochrome in the absence of a membrane carrier such as cells or lipid bilayered liposomes;
(iii) comparing the reference value obtained in (i) with the test value obtained in (ii) and, if at the same concentration of the ionizing compound, the fluorescent intensity of the potential-sensitive fluorochrome as obtained in (ii) is higher than the fluorescent intensity as obtained in (i) or if for at least two different concentrations of the ionizing compound added, the percent increase in fluorescent intensity as obtained in (ii) is higher than the percent increase as obtained in (i), selecting the test substance as a substance that enhances the percent change of fluorescent intensity of the potential-sensitive fluorochrome depending on the potential or ionic strength.

### ADVANTAGEOUS EFFECTS OF INVENTION

It has become clear that irrespective of whether an experiment is performed in a system that does not use a membrane carrier such as cells or lipid bilayered liposomes or in the conventional system which uses a membrane carrier such as cells or lipid bilayered liposomes, the sensitivity of fluorescence depending on a potential or ionic strength change from potential-sensitive fluorochromes can be increased by vitamin E and that fluorescent intensity of potential-sensitive fluorochromes depending on the potential or ionic strength change can be enhanced by both vitamin E and cholesterol.

### BRIEF DESCRIPTION OF DRAWINGS

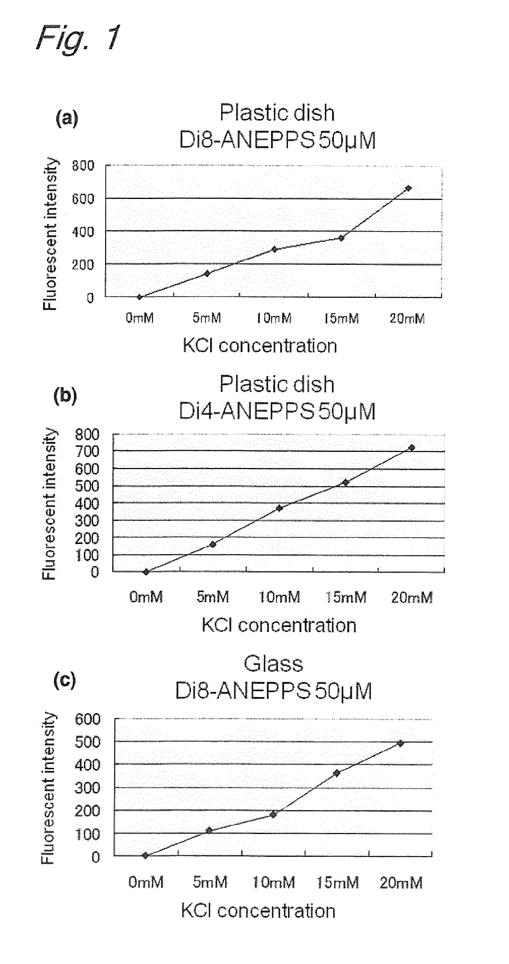

FIG. 1 is a set of graphs showing that potential-sensitive fluorochromes forming a solid phase on a substrate's surface emitted fluorescence at intensities proportional to the ionic strength under such conditions that there were no cells serving as a membrane carrier; obviously, irrespective of the substrate's material (glass or plastic) or the potential-sensitive fluorochrome's type, the fluorescent intensity increased in proportion to the concentration of the ionizing compound added (potassium chloride).

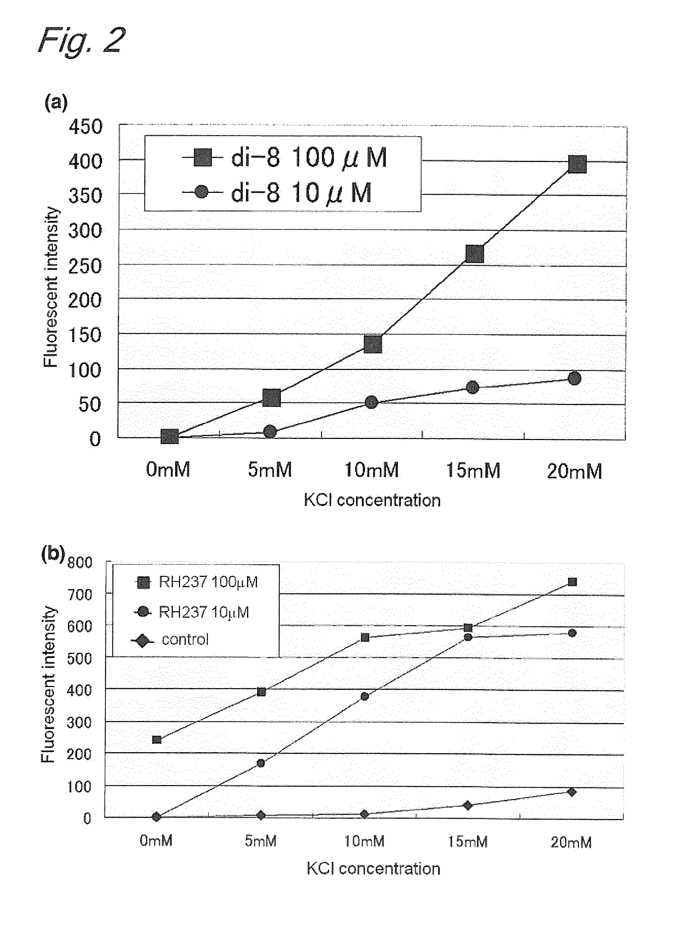

FIG. 2 is a set of graphs showing potential-sensitive fluorochromes forming a solid phase on a substrate's surface emitted fluorescence at intensities proportional to the ionic strength under such conditions that there were no cells serving as a membrane carrier; for Di8-ANEPPS (FIG. 2a) and RH237 (FIG. 2b), changes in fluorescent intensity occurred in proportion to the concentration of the ionizing compound added (potassium chloride).

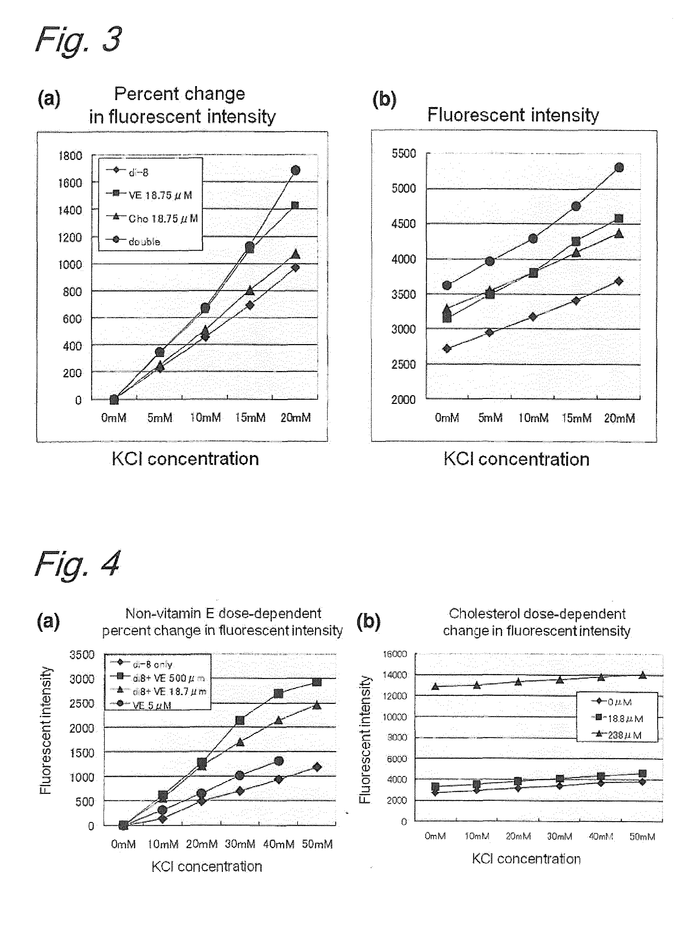

FIG. 3 is a set of graphs showing that vitamin E functions as a substance capable of increasing the potential-dependent percent change in fluorescent intensity of a potential-sensitive fluorochrome (i.e., a substance having a sensitizing action) and that both cholesterol and vitamin E function as an enhancer of potential-dependent fluorescent intensity of a potential-sensitive fluorochrome.
FIG. 4 is a set of graphs showing that vitamin E functions as a substance capable of increasing the potential-dependent percent change in fluorescent intensity of a potential-sensitive fluorochrome (i.e., a substance having a sensitizing action) and that both cholesterol and vitamin E function as an enhancer of potential-dependent fluorescent intensity of a potential-sensitive fluorochrome.

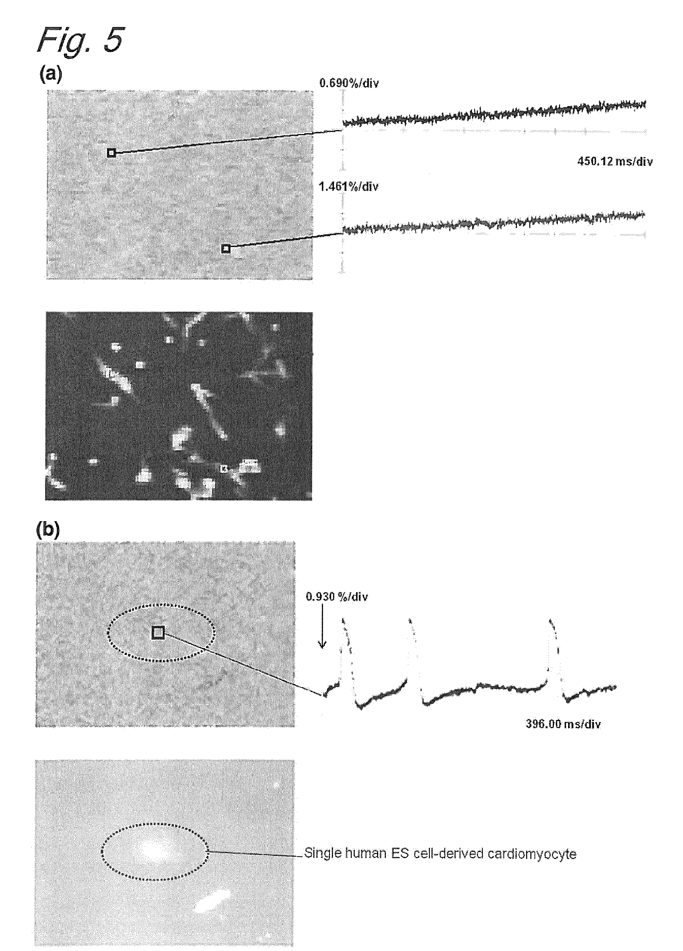

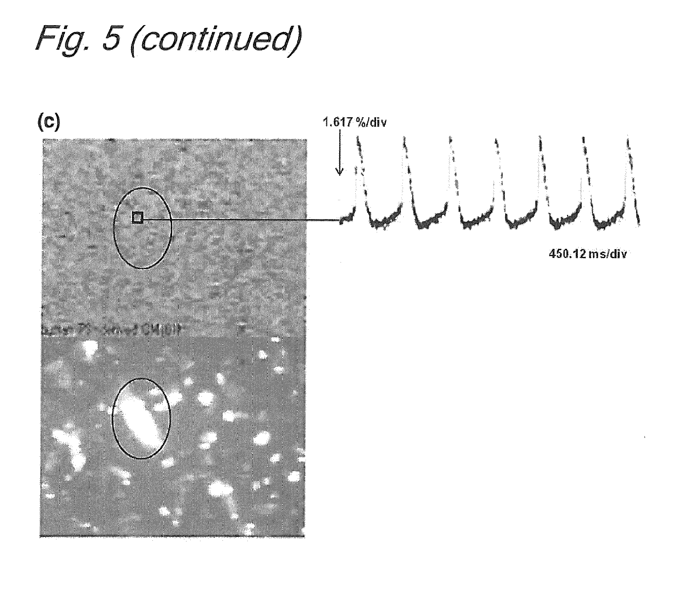

FIG. 5 is a set of photos showing that vitamin E and cholesterol can also exhibit a sensitizing or enhancing action for a potential-sensitive fluorochrome under such conditions that cells were used as a membrane carrier.

### DESCRIPTION OF EMBODIMENTS

Potential-sensitive fluorochromes are substances useful for measuring the activity potential of cardiomyocytes or neurons. Various substances hare heretofore been reported as potential-sensitive fluorochromes. The conventional methods which use potential-sensitive fluorochromes to analyze the characteristics of activity potential in terms of fluorescent intensity involve measuring the potential difference between the inside and outside of a cell membrane using a membrane structure (membrane carrier) such as cells or lipid bilayered liposomes. In these conventional methods, cells having autonomous electrogenic activity are not used; rather, a membrane carrier such as cells or lipid bilayered liposomes having no electrogenic activity is used and an ionizing compound is added from outside the cell to generate a potential difference across the cell membrane; the technique based on this approach has been commonly used as a quantitative analytical method.

Cells are used to perform potential measurement on potential-sensitive fluorochromes because potential-sensitive fluorochromes, which were initially intended to measure the potential difference across the cell membrane, have been specifically developed to acquire a higher ability to migrate towards the cell membrane in order to attain that objective.

In order to search for additives that would enable changes in activity potential to be detected with higher sensitivity, the present inventors first performed measurements in accordance with the conventional method using cells as a membrane carrier. When a greater amount of potential-sensitive fluorochrome was added with a view to enhancing the fluorescent intensity from the potential-sensitive fluorochrome, the fluorescent intensity on the cell culture dish rather than on the cells increased, namely, the fluorescent intensity of the background increased. This phenomenon suggests immobilization of the potential-sensitive fluorochrome on a surface of the cell culture dish as the substrate. Since it has heretofore been considered necessary that a potential-sensitive fluorochrome be subjected to experimentation after it is treated to form a solid phase on a membrane carrier such as cells or lipid bilayered liposomes, the possibility of immobilizing the potential-sensitive fluorochrome on a surface of a substrate such as plastics, glass, etc. has been a totally unexpected phenomenon.

Based on this finding, the present inventors measured changes in fluorescent intensity dependent on the concentration of an added ionizing compound (i.e., ionic strength) in accordance with the conventional method, except that a potential-sensitive fluorochrome was not treated to form a solid phase on a membrane carrier such as cells or lipid bilayered liposomes but allowed to adhere to a surface of a cell adherent substrate such as plastics or glass. As the result, the inventors found that even without the use of a membrane carrier such as cells or lipid bilayered liposomes, fluorescent intensity was enhanced in a manner dependent on the concentration of the ionizing compound (i.e., ionic strength). This has enabled convenient measurements of the fluorescent intensity of a potential-sensitive fluorochrome and the potential-dependent quantitative change in fluorescent intensity.

Thus, in one mode of the present invention, the inventors have shown that by a procedure comprising the steps of immobilizing a potential-sensitive fluorochrome on a surface of a substrate in a solution, adding an ionizing compound to the solution and measuring changes in the fluorescent intensity of the potential-sensitive fluorochrome depending on the potential or ionic strength change, there can be provided a method that measures the potential or ionic strength changes on the potential-sensitive fluorochrome even in the absence of a (membrane carrier such as cells or lipid bilayered liposomes.

The potential-sensitive fluorochrome as used herein may be any of the types that are generally available in the art concerned and a suitable one may be selected from among the following: styryl-based potential-sensitive fluorochromes comprising ANEPPSs, ANRPEQs and RHs; cyanine- or oxonol-based potential-sensitive fluorochromes comprising DiSC's, DiOC's, DiIC's, DiBAC's, and DiSBAC's; and rhodamine-derived potential-sensitive fluorochrome such as Rh 123, TMRM, and TMRE. In the present invention, it is more preferred to use styryl-based potential-sensitive fluorochromes comprising ANEPPSs, ANRPEQs and RHs, which are specifically exemplified by di-8-ANEPPS, di-4-ANEPPS, RH-237, RH-1691, di-5-ASP, RH-160, RH-421, RH-795, di-4-ANEPPDHQ, ANNINE-5, and ANNINE-6, and a preferred potential-sensitive fluorochrome may be selected from among these.

The ionizing compound to be added to the potential-sensitive fluorochrome immobilized on a substrate surface in a solution may be any substance that ionizes (changes into ions) in the solution. To state more specifically, it may be an ionizing compound that is solely composed of ions contained in a biological tissue fluid or an intracellular fluid, such as potassium, sodium, calcium, bicarbonate ion, chloride ion, hydroxyl ion, and ammonium ion. In the present invention, potassium chloride, calcium chloride or sodium chloride may typically be used as a preferred ionizing compound.

The surface of a substrate such as plastics or glass need not be given any special treatment; alternatively, it may be subjected to a surface treatment that allows for easy adhesion of cells. In whichever case, the substrate's surface is subsequently treated at a suitable concentration of a potential-sensitive fluorochrome (say, 100 µM or less in the case of ANEPPS) for a suitable period of time (say, 15 minutes in the case of ANEPPS) at a suitable temperature (say, 20 °C in the case of ANEPPS). The treated surface of the substrate is washed with a suitable aqueous solution at least three times to thereby remove the potential-sensitive fluorochrome that has not been immobilized on the surface.

As a further advantage of the present invention, by using the above-described method which measures the potential or ionic strength changes on the potential-sensitive fluorochrome in the absence of a (membrane carrier such as cells or lipid bilayered liposomes, the measurement of the potential or ionic strength changes on the potential-sensitive fluorochrome that was conventionally performed using a (membrane carrier such as cells or lipid bilayered liposomes became highly amenable to implementation in vitro. Hence, the present inventors adopted this method for the specific purpose of screening for substances that would enhance the change in fluorescent intensity of the potential-sensitive fluorochrome, as well as substances that would facilitate the detection of this change in fluorescent intensity. Thus, in another mode of the present invention, the inventors have shown that by using the above-described method, there can be provided a method of screening for a substance that modifies the fluorescent intensity of the potential-sensitive fluorochrome (namely, a substance that enhances fluorescent intensity or a substance that lowers it) as well as a substance that facilitates the detection of a change in fluorescent intensity (namely, a substance that facilitates the detection of an increased change of fluorescent intensity or a substance that facilitates the detection of a decreased change of fluorescent intensity).

Stated more specifically, there is provided a method for selecting a substance that modifies the percent change in fluorescent intensity of a potential-sensitive fluorochrome depending on the potential or ionic strength, comprising:

(i) immobilizing a potential-sensitive fluorochrome on a surface of a substrate in a solution, adding an ionizing compound to the solution, and measuring changes in the fluorescent intensity of the potential-sensitive fluorochrome depending on a potential or ionic strength change to thereby measure a reference value for the potential or ionic strength on the potential-sensitive fluorochrome in the absence of a membrane carrier such as cells or lipid bilayered liposomes;

(ii) immobilizing a potential-sensitive fluorochrome on a surface of a substrate in a solution, adding an ionizing compound and a test substance to the solution, and measuring changes in the fluorescent intensity of the potential-sensitive fluorochrome depending on a potential or ionic strength change to thereby measure a test value for the potential or ionic strength on the potential-sensitive fluorochrome in the absence of a membrane carrier such as cells or lipid bilayered liposomes;

(iii) comparing the reference value obtained in (i) with the test value obtained in (ii) and, if the fluorescent intensity of the potential-sensitive fluorochrome as obtained in (ii) is higher than the fluorescent intensity as obtained in (i) or if for at least two different concentrations of the ionizing compound added, the percent increase in fluorescent intensity as obtained in (ii) is higher than the percent increase as obtained in (i), selecting the test substance as a substance that modifies the percent change of fluorescent intensity of the potential-sensitive fluorochrome depending on the potential or ionic strength.

The term "modify [modifies]" as used herein in relation to the percent change of fluorescent intensity of a potential-sensitive fluorochrome depending on the potential or ionic strength may refer to either enhancing or lowering the percent change of interest. In the present invention, a substance that enhances the fluorescent intensity of the potential-sensitive fluorochrome and a substance that facilitates the detection of an increase in the fluorescent intensity are both preferred.

In the conventional screening method which used cells as the membrane carrier, it was difficult to determine whether the substance under screening would act on proteins such as ion channels present in the cells or would involve direct interaction with the potential-sensitive fluorochrome. Unlike this conventional method, the above-described screening method of the present invention does not use a membrane carrier such as cells or lipid bilayered liposomes, so it is possible to select a substance that directly interacts with the potential-sensitive fluorochrome.

The present inventors added a number of substances as examples of the test substance referred to in step (ii) of the above-described method and checked to see if each substance would enhance the fluorescent intensity of potential-sensitive fluorochromes or facilitate the detection of a change in their fluorescent intensity. As a result, they found that two substances, vitamin E and cholesterol, having different characteristics enhanced the fluorescent intensity of potential-sensitive fluorochromes. A close study of this action revealed that vitamin E had an action for enhancing the sensitivity of potential-sensitive fluorochromes whereas both vitamin E and cholesterol have an action for enhancing the fluorescent intensity of the potential-dependent fluorochromes.

This screening method, which adopts an experimental system that does not use a membrane carrier such as cells or lipid bilayered liposomes, is capable of picking up a change in fluorescent intensity that is based on the direct interaction between the potential-sensitive fluorochrome and the test substance. So it became clear that vitamin E and cholesterol, rather being assisted by the action of the membrane or the membrane's potential, had a direct action on the potential-sensitive fluorochrome to thereby enhance its sensitivity for surrounding ions, as well as its fluorescent intensity.

In addition, in view of the properties of vitamin E and cholesterol, it is easy to expect similar functions not only from vitamin E derivatives and cholesterol derivatives but also from liposoluble antioxidants including derivatives such as butylated hydroxytoluene, trolox, catechin and astaxanthin, compounds described in known documents (e.g., Advances in Drug Research, Vol. 28, 1996, pp. 65-138 and Toxicology, Vol. 180, 2002, pp. 151-167), and derivatives thereof.

In the case of screening for a substance that affects the fluorescent intensity and the potential-dependent change in fluorescent intensity, a potential-sensitive fluorochrome that is to form a solid phase may be added to a solution, which is then treated by the method described above. The present inventors tested this screening method to check for the relationship between the concentration of vitamin A or cholesterol and their sensitizing or enhancing action in connection with fluorescent intensity. As the result, they found that vitamin E, when used at concentrations of 500 µM to 5 µM, could enhance the sensitivity of potential-sensitive fluorochromes while at the same time enhancing their fluorescent intensity. The inventors also found that cholesterol, when used at concentrations of 500 µM to 5 µM, could enhance the fluorescent intensity of the potential-sensitive fluorochromes.

Using the sensitivity or fluorescent intensity enhancing substances thus obtained by the above-described screening method, the present inventors have further shown that changes in activity potential that occur in a population of ES cell- or iPS-cell derived cardiomyocytes or a single ES cell- or iPS-cell derived cardiomyocyte or in certain areas of such cardiomyocytes can be measured with higher sensitivities than possible in the conventional method. Specifically, the inventors have shown that the activity potential of cultured cardiomyocytes can be measured with higher sensitivity than possible in the conventional method by the procedure of bringing a potential-sensitive fluorochrome into contact with cardiomyocytes being cultured in a culture medium, adding vitamin E and/or cholesterol to the culture medium, and measuring changes in the fluorescent intensity of the potential-sensitive fluorochrome depending on a potential or ionic strength change.

To measure the activity potential of cardiomyocytes, the surface of a substrate such as plastics or glass is subjected to any suitable treatment for prompting cell adhesion and cardiomyocytes are subjected to adherent culture. To the adherent culture medium, a potential-sensitive fluorochrome is added so as to stain the cultured cells and vitamin E and/or cholesterol is also added at concentrations of 500 µM to 5 µM. In this case, vitamin E or cholesterol may be used independently or they may be used in combination.

For fluorescence assay, any type of fluorescent microscope that can be used in the art concerned may be applied in the present invention and a typical example is IX71 (OLYMPUS Corporation). In the Examples that follow, IX71 (OLYMPUS Corporation) was used as a fluorescent microscope and combined with a suitable light source unit such as a mercury lamp (OLYMPUS Corporation) or an LED assembly (OLYMPUS Corporation). For the purposes of capturing fluorescent images, imaging and numerical calculations, any models of analysis software for imaging and numerical calculations that can be used in the art concerned may be applied in the present invention. In the Examples that follow, the MiCAM02 system (Brainvision Inc.) was used.

The foregoing results, taken together, have shown that not only in the experimental system that does not involve the use of a membrane carrier such as cells or lipid bilayered liposomes but also in the conventional experimental system which uses a membrane carrier such as cells or lipid bilayered liposomes, vitamin E is able to enhance the sensitivity of potential-sensitive fluorochromes whereas both vitamin E and cholesterol are able to enhance fluorescent intensity of the potential-sensitive fluorochromes depending on the potential or ionic strength change. Thus, it has been shown that the present invention can provide a novel method for measuring changes in the fluorescent intensity of a potential-sensitive fluorochrome depending on the potential or ionic strength change, which comprises adding an ionizing compound to the potential-sensitive fluorochrome to confer a potential or ionic strength change and also adding vitamin E and/or cholesterol to enhance the potential or ionic strength change on the potential-sensitive fluorochrome.

### EXAMPLES

The present invention is described in greater detail by referring to the following Examples. It should, however, be noted that those Examples are illustrations of the present invention and are by no means intended to limit the same.

### Example 1: Construction of a method for measuring fluorescent intensity and potential-dependent change in fluorescent intensity using potential-sensitive fluorochromes that formed a solid phase-on a substrate surface

Described in Example 1 are a method of forming a solid phase of potential-sensitive fluorochromes on the surfaces of substrates such as transparent plastic or glass, and a method for measuring the fluorescent intensities of the potential-sensitive fluorochromes and the potential-dependent changes in their fluorescent intensity.

A potential-sensitive fluorochrome (Di8-ANEPPS or Di4-ANEPPS, both available from Invitrogen) was mixed in an amount of 50 µM in MEM-BASE medium (Sigma-Aldrich Corporation, St. Louis, Missouri, USA) containing fetal bovine serum (SAFC Biosciences, Lenexa, Kansas, USA) at a final concentration of 10% and the mixture was passed through a 0.22 µm syringe filter (Millipore, Massachusetts, USA) to prepare a potential-sensitive fluorochrome solution.

As 1 mL portion of this fluorochrome was added to each of a 3.5 cm plastic culture dish (BD, New Jersey, USA) and a 3.5 cm glass bottom dish (IWAKI, Asahi Glass Co., Ltd., Tokyo, Japan), followed by surface treatment at 20 °C for 15 minutes. The treated surfaces were washed at least three times with the same solution as described above excepted that it did not contain any potential-sensitive fluorochrome and that it had been warmed up to 37°C, thereby removing any part of the potential-sensitive fluorochrome that did not form a solid phase.

The medium was replaced by MEM-BASE conaining 10% fetal bovine serum supplemented with 50 µM of Mn-TBAP (Calbiochem, Merck KGaA, Darmstadt, Germany) and 992 µM of Trolox (Wako Pure Chemical Industries, Ltd., Osaka, Japan) and fluorescent signals were captured using the fluorescent microscopic system IX71 (Olympus Corporation, Tokyo, Japan) and a mercury lamp (Olympus Corporation, Tokyo, Japan) or an LED light source (Olympus Corporation, Tokyo, Japan), followed by imaging and numerical calculations using the MiCAM02 system (Brainvision Inc., Tokyo, Japan).

In order to provide potential changes in the potential-sensitive fluorochrome solution, a potassium chloride solution was added to the fluorochrome solution to give final concentrations of 0, 5, 10, 15 and 20 mM. Following 1-min standing after the addition of potassium chloride, the fluorescent intensity from the surface of each dish (where the potential-sensitive fluorochrome was present) was measured. The fluorescent intensity for each concentration of the fluorochrome was sampled at three different points on the dish and a standard deviation was determined from the average of the sampled intensities. The data were plotted to give graphs for the fluorescent intensity and the change in fluorescent intensity (FIG. 1).

In FIG. 1, (a) refers to the case where Di8-ANEPPS formed a solid phase on the surface of the plastic dish, (b) the case where Di4-ANEPPS formed a solid phase on the surface of the plastic dish, and (c) the case where Di8-ANEPPS formed a solid phase on the surface of the glass dish. From these results, it became clear that the fluorescent intensity increased in proportion to the concentration of the added ionizing compound (potassium chloride) independently of what material the substrate was made of (glass or plastic) or what was the type of the potential-sensitive fluorochrome used.

In addition, in order to determine the concentration range over which the potential-sensitive fluorochrome worked effectively, 10 µM or 100 µM of Di8-ANEPPS or RH237 (both available from Invitrogen) was applied to the surface of a 3.5 cm plastic culture dish and treated as described above to prepare a potential-sensitive fluorochrome solution; thereafter, a potassium chloride solution was added to the fluorochrome solution to give final concentrations of 0, 5, 10, 15 and 20 mM. The data obtained by performing subsequent treatments, measurements and processing as described above are plotted on graphs (FIG. 2).

In FIG. 2, (a) shows the data for the case where Di8-ANEPPS was used in amounts of 10 µM and 100 µM and (b) the data for the case where RH237 was used in amounts of 10 µM and 100 µM. From these results, it became clear that changes in the fluorescent intensity occurred in proportion to the concentration of the added ionizing compound (potassium chloride).

### Example 2: Screening for enhancers of florescent intensity and potential-dependent percent change in fluorescent intensity (sensitivity) using a potential-sensitive fluorochrome that formed a solid phase can a substrate surface

Described in this Example are the screening method for finding out substances that would enhance the fluorescent intensity of the potential-sensitive fluorochrome mentioned in Example 1 and the potential-dependent percent change in its fluorescent intensity (i.e., its sensitivity), as well as the methods of identifying the substances having the respective actions.

In accordance with the procedure described in Example 1, a solution of Di8-ANEPPS (100 µM) was prepared as a solution of potential-sensitive fluorochrome, to which 75 µM of vitamin E (Wako Pure Chemical Industries, Ltd.) or 75 µM of cholesterol (Wako Pure Chemical Industries, Ltd.) or a combination thereof was added. The potential-dependent quantitative change in fluorescent intensity and the potential-dependent fluorescent intensity were assayed as in Example 1 by using the method of adding the potassium chloride solution to give final concentrations of 0, 5, 10, 15 and 20 mM; the results are shown graphically in FIG. 3 for the vitamin E added group ("VE 18.75 µM"), the cholesterol added group ("Cho 18.75 µM"), the vitamin E + cholesterol added group ("double"), and the control group ("di-8").

In FIG. 3, (a) shows the measured values of the percent change in fluorescent intensity and (b) shows the measured values of fluorescent intensity; in each graph, ◆ refers to the control group (only Di8-ANEPPS was added), ■ the vitamin E added group, ▲ then cholesterol added group, and ● the vitamin E + cholesterol added group.

The percent change in fluorescent intensity as plotted in FIG. 3(a) in comparison with the case where no potassium chloride was added clearly shows that vitamin E has the sensitizing action for the potential-sensitive fluorochrome (i.e., enhancing its fluorescing sensitivity).

The increase in fluorescent intensity as compared at varying KCl concentrations in FIG. 3(b) showed that both vitamin E and cholesterol have the action for enhancing the fluorescent intensity of the potential-sensitive fluorochrome. In addition, the combined use of vitamin E and cholesterol not only had an additive effect on the increase of fluorescent intensity but also exhibited the vitamin E derived sensitizing action for the potential-sensitive fluorochrome (i.e., enhancing its fluorescing sensitivity or the potential-dependent percent change in fluorescent intensity). These results demonstrate the great superiority of the method disclosed herein as the way to screen for substances that would enhance the fluorescent intensities of potential-sensitive fluorochromes and/or substances that would increase the potential-dependent percent change in fluorescent intensity.

Furthermore, in order to determine the practically effective concentration ranges of cholesterol and vitamin E, 5 µM, 18.7 µM or 500 µM of vitamin E or 18.8 µM or 238 µM of cholesterol was added to 100 µM of Di8-ANEPPS in solution; each of the solutions was applied to the surface of a 3.5 cm plastic culture dish and a KCl solution was then added to the fluorochrome solutions to give final concentrations of 0, 10, 20, 30, 40 and 50 mM; the results of subsequent treatments and data processing are plotted on graphs (FIG. 4).

As it turned out, cholesterol enhanced the fluorescent intensity in a dose-dependent manner whereas vitamin E, being not dose-dependent in terms of either fluorescent intensity or percent change in fluorescent intensity, increased the fluorescent intensity by about 1.5 times and the percent change in fluorescent intensity by about 3 times as long as its concentration was within the range of 18.7 µM to 500 µM. It also became clear that vitamin E had an action for increasing the percent change in fluorescent intensity even when its concentration was as low as 5 µM.

### Example 3: Activity potential measurements of cardiomyocytes derived from human ES cells and human iPS cells

In this Example, the actions of vitamin E and cholesterol on the acquisition of activity potential from cardiomyocytes were studied; the cardiomyocytes had been differentiated from human ES cells and human iPS cells and subjected to enzymatic dissociated culture.

Human embryonic stem cells (ES cells) were obtained from Stem Cell Research Center, the Institute for Frontier Medical Sciences, Kyoto University (Embryonic Stem Cell Center sponsored by the National Bioresource Project). Human induced pluripotent stem cells (iPS cells) were obtained from the Center for iPS Cell Research and Application, Institute for Integrated Cell-Material Sciences, Kyoto University.

These human stem cells were allowed to remain undifferentiated by culture with the aid of mouse embryonic fibroblasts (MEF) that had been rendered inactive for proliferation by treatment with mitomycin C. To prepare the MEF cells, the fetus of an ICR mouse at day 14 of gestation (CLEA Japan, Inc.) was beheaded and disemboweled and thereafter disintegrated into discrete cells by the method described in WO 2006/022377.

The culture medium was F12/DMEM (1 : 1) (Sigma-Aldrich Corporation; Product No. D6421) which was supplemented with 20% KO-SERUM (GIBCO, Life Technologies Foundation, Maryland, USA), 1.6 mM L-glutamine, 0.1 mM nonessential amino acids (MEM), 0.1 mM β-mercaptoethanol (2ME; Sigma-Aldrich Corporation), 100 IU/ml penicillin, 100 µg/ml streptomycin sulfate, and 10 ng/ml recombinant human basic fibroblast growth factor (bFGF; PeproTech Inc., New Jersey, USA.) For subculturing, embryonic stem cell colonies were separated at 37 °C by 10-min treatment with 0.1% type III collagenase (Worthington Biochemical Corporation, New Jersey, USA.)

Subsequently, in order to separate MEF from the embryonic stem cells, cell masses (embryoid bodies, EB) were obtained on a mesh having a pore size of 40 µm. They were pure embryonic stem cell masses. For differentiation, 50-1000 embryonic stem cells per EB were cultured as embryoid bodies on a cell non-adherent bacterial dish (AGC TECHNO GLASS CO., LTD., Chiba, Japan; sterilized Petri dish) for a total of 15-30 days until they were differentiated into embryoid bodies containing cardiomyocytes.

The cardiomyocytes were disintegrated by the method described in WO 2006/022377. Specifically, the embryoid body was treated with collagenase and trypsin to make discrete single cells. These cells were suspended in 10% serum containing αMEM (Sigma-Aldrich Corporation) and seeded on a 3.5 cm plastic culture dish (BD) and a 3.5 cm glass bottom dish (IWAKI, Asahi Glass Co., Ltd.), each having been surface-coated with 0.001% fibronectin (Sigma-Aldrich Corporation) at 37 °C for an hour. The adhering cardiomyocytes were beating autonomously.

Activity potential measurements on these cardiomyocytes were made in the absence of vitamin E and cholesterol. In other experiments, the cultured cells were treated with a potential-sensitive fluorochrome and 18.75 µM of vitamin E and/or 18.75 µM of cholesterol and then the temporal change in fluorescent intensity was measured. This treatment enabled acquisition of activity potentials derived from a single cell as well as part of a single cell

### (FIG. 5.)

FIG. 5(a) shows the result of measurements on isolated human ES cell derived cardiomyocytes in the absence of vitamin E and cholesterol; FIG. 5(b) shows the activity potential acquired from an isolated human ES cell derived cardiomyocyte; and FIG. 5(c) shows the activity potential acquired from an isolated human iPS cell derived cardiomyocyte.

As it turned out, activity potential waveforms that had been impossible to detect from single ES cell derived cardiomyocytes in the absence of vitamin E or cholesterol became observable upon addition of these substances. It also became clear that vitamin E and cholesterol which had the action for sensitizing or enhancing the potential-sensitive fluorochrome in the absence of cells as the membrane carrier could also exhibit the same action even when cells were used as the membrane carrier.

## Claims

1. A method for measuring changes in the fluorescent intensity of a potential-sensitive fluorochrome depending on a potential or ionic strength change, which comprises adding an ionizing compound to a potential-sensitive fluorochrome to confer a potential or ionic strength change and also adding vitamin E and/or cholesterol to enhance the potential or ionic strength change on the potential-sensitive fluorochrome.

2. The method according to claim 1, wherein 500 µM - 5 µM of vitamin E is used either alone or in combination with 500 µM - 5 µM of cholesterol.

3. The method according to claim 1 or 2, wherein the potential-sensitive fluorochrome is selected from the group consisting of anellated hemicyanine-based potential-sensitive fluorochromes comprising ANNINEs, biaryl hemicyanine-based potential-sensitive fluorochromes comprising BNBIQs, and styryl hemicyanine-based potential-sensitive fluorochromes comprising ANEPPSs, ANRPEQs and RHs.

4. The method according to claim 3, wherein the potential-sensitive fluorochrome is selected from the group consisting of di-8-ANEPPS, di-4-ANEPPS, RH-237, RH-1691, di-5-ASP, RH-160, RH-421, RH-795, di-4-ANEPPDHQ, ANNINE-5, and ANNINE-6.

5. The method according to any one of claims 1-4, wherein the ionizing compound is selected from the group consisting of potassium chloride, calcium chloride, and sodium chloride.

6. A method for measuring the activity potential of cultured cardiomyocytes, which comprises bringing a potential-sensitive fluorochrome into contact with cardiomyocytes being cultured in a culture medium, adding vitamin E and/or cholesterol to the culture medium, and measuring changes in fluorescent intensity of the potential-sensitive fluorochrome depending on a potential or ionic strength change.

7. The method according to claim 6, wherein the cultured cardiomyocytes are primary cultured cardiomyocytes, embryonic stem cell derived cardiomyocytes, a single embryonic stem cell derived cardiomyocyte, induced pluripotent stem cell (iPS cell) derived cardiomyocytes, or a single induced pluripotent stem cell (iPS cell) derived cardiomyocyte.

8. The method according to claim 6 or 7, wherein 500 µM - 5 µM of vitamin E is used either alone or in combination with 500 µM - 5 µM of cholesterol.

9. The method according to any one of claims 6 to 8, wherein the potential-sensitive fluorochrome is selected from the group consisting of anellated hemicyanine-based potential-sensitive fluorochromes comprising ANNINEs, biaryl hemicyanine-based potential-sensitive fluorochromes comprising BEBIQs, and styryl hemicyanine-based potential-sensitive fluorochromes comprising ANEPPSs, ANRPEQs and RHs.

10. The method according to claims 9, wherein the potential-sensitive fluorochrome is selected from the group consisting of di-8-ANEPPS, di-4-ANEPPS, RH-237, RH-1691, di-5-ASP, RH-160, RH-421, RH-795, di-4-ANEPPDHQ, ANNINE-5, and ANNINE-6.

11. A method for measuring potential or ionic strength changes on a potential-sensitive fluorochrome in the absence of a membrane carrier, which comprises immobilizing a potential-sensitive fluorochrome on a surface of a substrate in a solution, adding an ionizing compound to the solution to confer a potential or ionic strength change, and measuring changes in the fluorescent intensity of the potential-sensitive fluorochrome depending on a potential or ionic strength change.

12. The method according to claim 11, wherein the substrate is plastic or glass.

13. The method according to claim 11 or 12, wherein the potential-sensitive fluorochrome is selected from the group consisting of anellated hemicyanine-based potential-sensitive fluorochromes comprising ANNINEs, biaryl hemicyanine-based potential-sensitive fluorochromes comprising BNBIQs, and styryl hemicyanine-based potential-sensitive fluorochromes comprising A-NEPPSs, ANRPEQs and RHs.

14. The method according to claims 13, wherein the potential-sensitive fluorochrome is selected from the group consisting of di-8-ANEPPS, di-4-ANEPPS, RH-237, RH-1691, di-5-ASP, RH-160, RH-421, RH-795, di-4-ANEPPDHQ, ANNINE-5, and ANNINE-6.

15. The method according to any one of claims 11 to 14, wherein the ionizing compound is selected from the group consisting of potassium chloride, calcium chloride, and sodium chloride.

16. A method for selecting a substance that modifies the percent change in the fluorescent intensity of a potential-sensitive fluorochrome depending on the potential or ionic strength, comprising:
(i) immobilizing a potential-sensitive fluorochrome on a surface of a substrate in a solution, adding an ionizing compound to the solution, and measuring a change in the fluorescent intensity of the potential-sensitive fluorochrome depending on a potential or ionic strength change to thereby measure a reference value for the potential or ionic strength on the potential-sensitive fluorochrome in the absence of a membrane carrier;
(ii) immobilizing a potential-sensitive fluorochrome on a surface of a substrate in a solution, adding an ionizing compound and a test substance to the solution, and measuring changes in the fluorescent intensity of the potential-sensitive fluorochrome depending on a potential or ionic strength change to thereby measure a test value for the potential or ionic strength on the potential-sensitive fluorochrome in the absence of a membrane carrier;
(iii) comparing the reference value obtained in (i) with the test value obtained in (ii) and, if at the same concentration of the ionizing compound, the fluorescent intensity of the potential-sensitive fluorochrome as obtained in (ii) is higher than the fluorescent intensity as obtained in (i) or if for at least two different concentrations of the ionizing compound added, the percent increase in fluorescent intensity as obtained in (ii) is higher than the percent increase as obtained in (i), selecting the test substance as a substance that modifies a percent change of the fluorescent intensity of the potential-sensitive fluorochrome depending on the potential or ionic strength.

17. The method according to claim 16, wherein the test substance that enhances a percent change of the fluorescent intensity of the potential-sensitive fluorochrome depending on the potential or ionic strength is selected.

18. The method according to claim 17, wherein the test substance selected is vitamin E or cholesterol.

19. The method according to any one of claims 16 to 18, wherein the substrate is plastic or glass.

20. The method according to any one of claims 16 to 19, wherein the potential-sensitive fluorochrome is selected from the group consisting of anellated hemicyanine-based potential-sensitive fluorochromes comprising ANNINEs, biaryl hemicyanine-based potential-sensitive fluorochromes comprising BNBIQs, and styryl hemicyanine-based potential-sensitive fluorochromes comprising ANEPPSs, ANRPEQs and RHs.

21. The method according to claim 20, wherein the potential-sensitive fluorochrome is selected from the group consisting of di-8-ANEPPS, di-4-ANEPPS, RH-237, RH-1691, di-5-ASP, RH-160, RH-421, RH-795, di-4-ANEPPDHQ, ANNINE-5, and ANNINE-6.

22. The method according to any one of claims 16 to 21, wherein the ionizing compound is selected from the group consisting of potassium chloride, calcium chloride, and sodium chloride.
